# EUROPEAN PATENT SPECIFICATION

(11) **EP 0 794 761 B1**
(45) Date of publication and mention of the grant of the patent: **25.07.2001**
(21) Application number: 95943173.5
(22) Date of filing: 15.12.1995
(51) Int. Cl.: A61K 7/16, A61K 7/50

(54) **DENTAL HYGIENE COMPOSITIONS**
ZAHNHYGIENE - PRÄPARATE
COMPOSITIONS D'HYGIENE DENTAIRE

(30) Priority: 22.12.1994 DE 4446050
(43) Date of publication of application: 17.09.1997
(73) Proprietor: SmithKline Beecham Consumer Healthcare GmbH, 77815 Bühl (DE)
(72) Inventor: STILLER, Sigrid, SmithKline Beecham GmbH, 77815 Bühl (DE); KAWA, Gertrud, SmithKline Beecham GmbH, 77815 Bühl (DE)
(74) Representative: Reeves, Julie Frances
(86) International application number: EP9505059
(87) International publication number: WO9619189

(56) References cited:
- WO-A-92/07550
- WO-A-95/02392
- GB-A- 2 112 642
- US-A- 3 729 553

## Description

The present invention relates to a 2-phase dental hygiene product in paste form which releases carbon dioxide, which contains a bicarbonate or a carbonate and a surfactant.

Dental hygiene products in paste form have been commonly used in most households for decades. The cleaning effect obtained with them is generally based on a combination firstly of mechanical action on the tooth surface by means of the movement of the toothbrush or by means of the movement of the cleaning materials present in the paste which this causes, and secondly of the fine-pored foam formed by the intermediary of a surfactant, which serves to remove contaminants situated on the surface of the tooth and as a transporting agent for the active ingredients. In order to promote this foam formation, which is desirable for the purpose of effecting a particularly thorough cleaning of the teeth, dental hygiene products which are customary in the prior art have recently contained a bicarbonate or carbonate, which is subjected during the cleaning operation to a partial neutralisation by the acid present in oral saliva which is formed by the fermentation of carbohydrates from plaque bacteria. Carbon dioxide is thereby evolved in the course of a plurality of equilibrium reactions which are linked to each other, which results in the formation of foam being more intensive than when this only occurs due to the surfactant.

Thus the prior art *"baking soda"* toothpaste contains a sodium bicarbonate additive, for example, which gives rise to foam formation during the teeth cleaning procedure. However, because when prior art dental hygiene products of this type are used it is solely the weakly acid oral saliva which is available for the neutralisation reaction described above, neutralisation of the bicarbonate takes place only relatively slowly, and moreover is generally incomplete. This in turn is inevitably associated with a correspondingly delayed or decreased formation of foam and therefore with a cleaning effect which is to be considered as unsatisfactory. Furthermore, the addition of bicarbonate in a not inconsiderable concentration, which moreover is only decomposed slowly and gradually in the course of the cleaning procedure, can result in a prior art toothpaste of this type having a bitter, salty taste which is perceived as disagreeable by the user. This taste, which is perceived as disturbing by the user, can lead in particular to the teeth cleaning procedure often being discontinued too early, which additionally undermines the intended cleaning effect.

In GB -B-2 112 642 (Colgate) a dual component dental hygiene product is described which contains in one compartment at least 15% by weight of sodium bicarbonate, preferably 20 to 40% by weight and in the other compartment a reactive acid compound. The only example in this patent is of a toothpaste comprising 20% by weight sodium bicarbonate.

WO 95/03292 (Unilever) describes an effervescent oral care composition comprising a first composition containing an alkali metal carbonate and a second composition comprising an acidic compound characterised in that the bicarbonate is present in an amount of 2.5 to 10% by weight of the first composition. This weight range is chosen to significantly lessen the salty taste that the bicarbonate imparts.

There is therefore a need for a dental hygiene product which avoids or circumvents the disadvantages and limitations described above of prior art dental hygiene products whilst at the sme time produces an immediate and intensive formation of foam after the commencement of the teeth cleaning procedure, so as to be able to optimise the cleaning effect. In particular, a dental hygiene product which is improved compared with the prior art described above should contain no components which could be associated with taste sensations which are subjectively perceived as disagreeable and disturbing by the user.

We have since found that by incorporating a specific amount of bicarbonate or carbonate into a two phase formulation a more acceptable product is obtained that combines an acceptable taste profile with the required foaming characteristics.

According to the present invention there is provided a dental hygiene product in paste form which releases carbon dioxide and which contains a basic phase which contains a bicarbonate or carbonate and an acidic phase which contains a pharmacologically compatible, organic or inorganic proton donor component, which is in the form of two separate phases which are first brought into contact with each other after the dental hygiene product has emerged from the dispenser characterised in that the content of bicarbonate or carbonate in the basic phase is from 10 to 15 weight percent, and it contains a polyphosphate and Xylitol.

According to the invention, the bicarbonate or carbonate present in the basic phase is preferably the bicarbonate of an alkali or alkaline earth metal or a mixture of the same. The bicarbonates and/or carbonates of the elements Na, K, Ca and Mg are particularly preferred. In particular, the bicarbonate or carbonate is sodium bicarbonate or potassium bicarbonate. The bicarbonate or carbonate advantageously has an average particle size of 20 *µ*m to 200 *µ*m, preferably of 30 *µ*m to 100 *µ*m, and particularly of 40 *µ*m to 63 *µ*m.

In this manner it is ensured that the bicarbonate or carbonate additive which is present is rapidly and completely neutralised, directly after the commencement of the teeth cleaning procedure, with the release of CO₂, which firstly rules out disagreeable taste sensations on the part of the user from the outset and secondly gives rise to the rapid and efficient formation of a microfine foam due to the carbon dioxide released. By this means it is ensured that all regions of the surface of the teeth, including in particular even those sections which are only accessible with difficulty or are not accessible at all with conventional, prior art dental hygiene products, are contacted substantially uniformly by the toothpaste during the cleaning procedure. As a result of the transport effect based on an intensive formation of foam such as this, the distribution of the active ingredient obtainable during the teeth cleaning procedure is optimised, particularly the distribution of the polyphosphate which is preferably present, which firstly impedes the renewed build-up of tartar, due to chelating reactions, and secondly loosens deposits on the tooth surface which have a brownish coloration due to the consumption of coffee, tea or nicotine. This leads to the teeth having a whiter appearance with a bleaching process taking place. Moreover, the distribution of fluoride ions, which are crucial for oral prophylaxis, is optimised due to the microfine foam.

The two aforementioned phases of the dental hygiene product according to the invention are disposed in a supply receptacle in a manner such that they first come into contact after their emergence from the dispenser, preferably in the form of at least two superimposed strips or in the form of a coaxial double or multiple length. In this respect the acidic phase either forms the core of a double or multiple length or the bottom strip of a layer arrangement. By providing the acidic phase as the core of a coaxial length, with the basic phase as the outer layer, or the basic phase as the lower layer of superimposed strips, the advantage is achieved that the basic phase rather than the acidic phase is the first phase to contact the mouth of the user. In this way the user tastes the mild taste of the basic phase rather than the more sour taste of the basic phase, and the acid phase does not contact the mouth of the user until it has partly or wholly mixed with the basic phase and become neutralised. A coaxial length with the the acidic phase as the core is preferred.

A supply receptacle characterised by containing the dental hygiene product of the invention, and capable of dispensing the said product in a stream in the form of such superimposed strips or particularly in such a coaxial length is novel and is a further feature of this invention.

Such a supply receptacle will normally comprise separate respective reservoirs for the two phases, separate respective pump means for the two phases, and a dispensing nozzle with channels constructed to guide the flow of dispensed product in such a stream. Constructions of such a supply receptacle, and its reservoirs, pumps and nozzle suitable for the viscous product of this invention, and capable of dispensing it at a suitable rate, will be apparent to those skilled in the art. For example some suitable known constructions of supply receptacle which can dispense such two-phase products in coaxial lengths are disclosed in WO 93/04940 and EP 0202359A.

The two phases thus first come into contact with each other directly before the dental hygiene product according to the invention is used, but are then mixed very intimately with each other, which results in the desire, maximum rate of neutralisation of the added bicarbonate or carbonate. Moreover this rapid neutralisation causes a tingling sensation on the tongue and at the edges of the gums, which is due to the spontaneous formation of foam and which is perceived as pleasant by the user. This leads to a very pleasant sensation of cleanliness in the mouth. Since a negative taste sensation on the part of the user thus no longer appears, but instead he subjectively perceives the astringent and stimulating effect which a dental hygiene product according to the invention exerts on the gums as being markedly pleasant and relaxing, he moreover does not see himself, in contrast to the dental hygiene products known from the prior art, as having to terminate the cleaning procedure as rapidly as possible. This in turn encourages a greater degree of thoroughness of the teeth cleaning procedure which is thereby effected.

The dental hygiene product according to the invention also contains in the basic phase a polyphosphate, which firstly impedes the renewed build-up of tartar, due to chelating reactions, and secondly loosens deposits on the tooth surface which have a brownish coloration due to the consumption of coffee, tea or nicotine. This leads to the teeth having a whiter appearance without a bleaching process taking place. Moreover, in association with this polyphosphate which the product preferably contains, a synergistic cleaning effect occurs, due to the enhanced formation of foam, which results in a particularly smooth surface of the teeth which can be felt with the tongue. According to the invention, this polyphosphate is preferably sodium tripolyphosphate or potassium tripolyphosphate. It is most preferably sodium tripolyphosphate (pentasodium tripolyphosphate, Na₅P₃O₁₀; molecular weight 368 g/mole), advantageously with a particle size of 45 *µ*m to 100*µ*m, and particularly 45 *µ*m.

The content of polyphosphate in the basic phase of a preferred embodiment of the dental hygiene product according to the invention is advantageously 3 to 50 weight percent.

The acidic and the basic phases may be combined together in any volumetric ratio that provides a dental hygiene product according to the invention, however the acidic and basic phases are advantageously combined with each other in a volumetric ratio of 1:1.

It has been found that when a polyphosphate, for example sodium tripolyphosphate is added to the formulation there is a significant increase in the viscosity of the product leading to an unacceptably solid product. We have found that by incorporating the humectant Xylitol into the basic phase of the formulation the dramatic increase in viscosity levels can be overcome. Xylitol is either used singly or in combination with other humectants. Preferably the Xylitol is used singly and in the basic phase of the formulation. The content of Xylitol used in a dental hygiene product according to the invention is 1.0 to 50 weight percent, preferably 5.0 to 40 weight percent, and particularly 20 weight percent (for the basic phase of the formulation).

The viscosity of the acid and of the basic phase of a dental hygiene product according to the invention is thus adjusted in each case so that it is 80,000 to 150,000 mPa.s, measured with a Type DV-11+ Brookfield viscometer using a TD spindle at 10 revolutions per minute. The viscosities of the two phases advantageously do not differ greatly from each other; however they do not have to be absolutely identical

In addition, a dental hygiene product according to the invention contains one or more surfactants, advantageously from the group comprising anionic and/or nonionic and/or amphoteric surfactants. Sarcosinates and/or taurates and/or C₁₂-C₁₈ alkyl sulphates, most preferably C₁₂-C₁₈ alkyl sulphates, particularly sodium lauryl sulphate, are preferably used from the group comprising anionic surfactants. Alkyl saccharide ethers or other nonionic surfactants which contain a polyol group and/or a polyalkylene group, most preferably C₈-C₁₀ alkyl polyglucosides, particularly C₁₂-₁₆ alkyl polyglucosides, are used from the group comprising nonionic surfactants. Betaines, particularly lauramido- and/or cocamidopropyl betaines, are preferably used from the group comprising amphoteric surfactants.

The acidic and/or the basic phase of a dental hygiene product according to the invention advantageously additionally contains one or more of the following listed constituents a.) to i.) in each case:
a.) a moisture-retaining agent, particularly sorbitol and/or glycerine;
b.) a thickening agent, preferably xanthan gum and/or hydroxypropyl methyl cellulose and or hydroxyethyl cellulose and/or gum arabic and/or alginates, polyacrylates and gum tragacanth or mixtures of the same and/or thickening precipitated hydrated silica with an average particle size of 1 to 20 *µ*m, preferably of 1 to 15 *µ*m, preferably of 1 to 15 *µ*m, and advantageously with a specific surface of 50 to 250 m²/g, preferably of 100 to 200 m²/g, and particularly of 190 m²/g (determined by the BET method; DIN 66131);
   particularly xanthan gum and/or hydroxypropyl methyl cellulose and/or hydroxyethytl cellulose;
c.) a material with an abrasive action, preferably based on SiO₂; particularly abrasive precipitated hydrated silica with little thickening effect with an average particle size of 1 to 20 *µ*m, preferably of 1 to 15 *µ*m, and advantageously with a specific surface of 50 to 100 m2/g, preferably of 70 to 90 m2/g, and particularly of 80 m2/g (determined by the BET method; DIN 66131);
d.) a white pigment, preferably, preferably TiO₂; advantageously with an average particle size of 20 to 80*µ*m, preferably of 30 to 70 *µ*m and particularly of 40 to 60 *µ*m;
e.) sweetening agents, preferably acesulpam-K and/or neohesperidine and/or sodium cyclamate and/or sodium saccharin, particularly sodium saccharin;
f.) NaOH, in order to adjust the pH;
g.) colorants;
h.) flavourings;
i.) water.

The content of moisture retaining agents a.) in the acidic phase in a dental hygiene product according to the invention is advantageously 10 to 80 weight percent, preferably 20 to 70 weight percent, and particularly 60 weight percent. The content of moisture retaining agents a.) in the *basic* phase in a dental hygiene product according to the invention is advantageously within the range of 5 to 70 weight percent, particularly 10 to 50 weight percent.

The content of thickening agents b.) in the acid phase and/or in the basic phase of a dental hygiene product according to the invention is preferably 0.5 to 15 weight percent, and particularly 2.5 weight percent, in each case.

The content of abrasive material c.) in the acidic phase and/or in the basic phase of a dental hygiene product according to the invention is advantageously 8 to 20 weight percent, preferably 10 to 20 weight percent and particularly 12 to 16 weight percent, in each case.

The pharmacologically compatible, organic or inorganic, proton donor component contained in the acid phase of a dental hygiene product according to the invention is preferably citric acid, tartaric acid, raceme acid, succinic acid, lactic acid, malic acid or orthophosphoric acid or a mixture of the same; in particular it is citric acid, tartaric acid and/or malic acid, most preferably citric acid and/or tartaric acid.

The material content of the pharmacologically compatible proton donor component present in the acidic phase of a dental hygiene product according to he invention preferably corresponds to the neutralisation equivalent of the bicarbonate or carbonate present in the basic phase. For example, at a content of 15 weight percent sodium bicarbonate in the basic phase the corresponding content of citric acid in the acidic phase is thus preferably 6 weight percent.

According to a preferred embodiment, the acidic and/or basic phase of a dental hygiene product according to the invention additionally contains one or more water-soluble inorganic or organic fluorine compounds, preferably sodium fluoride, tin fluoride, bis(hydroxyethyl)-aminopropyl-N-hydroxyethyloctadecylaminodihydrofluoride, cetyl aminohydrofluoride and/or oleyl aminohydrofluoride or mixtures of the same, particularly sodium fluoride. In practice, the term *aminofluoride* is also commonly used as a collective term for organic fluorides which are preferably used. The Xylitol present may also offer some protection against caries.

The fluoride ion concentration used within the scope of an embodiment of this type depends on the age of the potential user. For children up to six years old it is 250 ppm to 500 ppm; for children from six years old and young persons it is 500 to 1000 ppm and for adults it is 900 to 1450 ppm, particularly 1200 ppm.

The dental hygiene product according to the invention will now be described with reference to the following representative examples.

### Example 1:

The two phases of a preferred embodiment of the dental hygiene product according to the invention have the composition given below:

| | *Basic phase* | *Acidic phase* |
|---|---|---|
| | (weight %) | (weight %) |
| sorbitol | 30.000 | 60.000 |
| water | 22.925 | 13.275 |
| PEG 6 | 2.000 | 2.000 |
| xanthan gum*) | 0.700 | 1.000 |
| sodium hydroxide (50 % solution in H₂O) | 0.900 | - |
| citric acid | - | 6.000 |
| sodium bicarbonate | 15.000 | - |
| sodium tripolyphosphate | 10.000 | - |
| sodium saccharin | 0.250 | 0.250 |
| sodium fluoride | 0.275 | 0.275 |
| SiO₂ (Sident 12 DS® )**) | 14.000 | 12.000 |
| SiO₂ (Sident 22 DS® )***) | - | 2.500 |
| flavouring | 1.600 | - |
| sodium lauryl sulphate | 1.700 | 1.700 |
| sodium benzoate | 0.100 | |
| titanium dioxide | - | 1.000 |
| FD&C blue No. 1 (1 %) | 0.550 | - |

| | | |
|---|---|---|
| *) as a thickening agent. | | |
| **) as a material with an abrasive action; with a specific surface of 80 m²/g (determined by the BET method, DIN 66131). | | |
| ***) a thickening hydrated silica with a specific surface of 190 m²/g (determined by the BET method, DIN 66131). | | |

### Example 2:

The two phases of another preferred embodiment of the dental hygiene product according to the invention have the composition given below:

| | *Basic phase* | *Acidic phase* |
|---|---|---|
| | (weight %) | (weight %) |
| sorbitol | 40.000 | 60.000 |
| water | 22.325 | 13.275 |
| PEG 6 | 2.000 | 2.000 |
| xanthan gum*) | 1.300 | 1.000 |
| sodium hydroxide (50 % solution in H₂O) | 0.900 | - |
| citric acid | - | 6.000 |
| sodium bicarbonate | 15.000 | - |
| sodium tripolyphosphate | - | - |
| sodium saccharin | 0.250 | 0.250 |
| sodium fluoride | 0.275 | 0.275 |
| SiO₂ (Sident 12 DS® )**) | 14.000 | 12.000 |
| SiO₂ (Sident 22 DS® )***) | - | 2.500 |
| flavouring | 1.600 | - |
| sodium lauryl sulphate | 1.700 | 1.700 |
| sodium benzoate | 0.100 | - |
| titanium dioxide | - | 1.000 |
| FD&C blue No. 1 (1 %) | 0.550 | - |

| | | |
|---|---|---|
| *) as a thickening agent | | |
| **) as a material with an abrasive action; with a specific surface of 80 m²/g (determined by the BET method, Din 66131). | | |
| ***) a thickening hydrated silica with a specific surface of 190 m²/g (determined by the BET method, DIN 66131). | | |

### Example 3:

### Effervescent toothpaste

| | *Basic phase* | *Acidic phase* |
|---|---|---|
| | (% w/w) | (% w/w) |
| sorbitol | - | 60.00 |
| water | 38.05 | - |
| xylitol | 20.00 | - |
| sodium hydroxide (50 %solution in H₂O) | 0.90 | - |
| sodium bicarbonate | 12.50 | - |
| sodium tripolyphosphate | 10.00 | - |
| sodium fluoride | 0.30 | 0.30 |
| sodium saccharin | 0.25 | 0.30 |
| titanium dioxide | 1.00 | - |
| SiO₂ (Sident 9) | 10.00 | 10.00 |
| SiO₂ (Sident 22 S) | 1.50 | 8.00 |
| PEG-6 | 2.00 | 2.00 |
| sodium lauryl sulphate | 1.50 | 1.50 |
| xanthan gum*) | 1.00 | 0.90 |
| tartaric acid | - | 2.40 |
| flavouring | 1.00 | 0.40 |
| colour (1 %) | - | 0.05 |

| | | |
|---|---|---|
| *) as a thickening agent | | |

### Viscosity of Basic and Acid phases:

Brookfield, TD, 10 rpm 70,000 - 100,000 mPas
Model RVDV II +
pH _{20°}8.9 - 9.5.

## Claims

1. A dental hygiene product in paste form which releases carbon dioxide, and which contains a basic phase which contains a bicarbonate or carbonate and an acidic phase which contains a pharmacologically compatible organic or inorganic proton donor component, which is in the form of two separate phases which are first brought into contact with each other after the dental hygiene product has emerged from the dispenser characterised in that the content of bicarbonate or carbonate in the basic phase is from 10 to 15 weight percent and it contains a polyphosphate and Xylitol.

2. A dental hygiene product according to claim 1 characterised in that the content of polyphosphate in the basic phase is 3 to 50 weight percent, preferably 5 to 40 weight percent, and particularly 5 to 20 weight percent.

3. A dental hygiene product according to claim 2 characterised in that the content of Xylitol in the basic phase is 1.0 to 50 weight percent, preferably 5 to 40 weight percent, and particularly 20 weight percent.

4. A dental hygiene product according to any one of claims 1 to 3, characterised in that the bicarbonate or carbonate is the bicarbonate of an alkali or alkaline earth metal or a mixture of the same.

5. A dental hygiene product according to any one of claims 1 to 4, characterised in that the pharmacologically compatible proton donor component is an organic or inorganic acid, preferably tartaric acid, citric acid, racemic acid, succinic acid, lactic acid, malic acid or orthophosphoric acid or a mixture of the same, particularly tartaric acid, citric acid or malic acid or a mixture of the same, most preferably tartaric acid or citric acid or a mixture of the same.

6. A dental hygiene product according to any one of claims 1 to 5, characterised in that the acidic and/or the basic phase additionally contains one or more of the following listed constituents a.) to i.) in each case:
a.) a moisture-retaining agent, particularly sorbitol and/or glycerine;
b.) a thickening agent, preferably xanthan gum and/or hydroxypropyl methyl cellulose and/or hydroxyethyl cellulose and/or gum arabic and/or alginates, polyacrylates and gum tragacanth or mixtures of the same and/or thickening precipitated hydrated silica with an average particle size of 1 to 20 *µ*m, preferably of 1 to 15 *µ*m, and advantageously with a specific surface of 50 to 250 m²/g, preferably of 100 to 200 m²/g, and particularly of 190 m²/g (determined by the BET method; DIN 66131);
particularly xanthan gum and/or hydroxypropyl mediyl cellulose and/or hydroxyethyl cellulose;
c.) a material with an abrasive action, preferably based on SiO₂; particularly abrasive precipitated hydrated silica with little thickening effect with an average particle size of 1 to 20 *µ*m, preferably of 1 to 15 *µ*m and advantageously with a specific surface of 50 to 100 m²/g, preferably of 70 to 90 m²/g, and particularly of 80 m²/g (determined by the BET method; DIN 66131);
d.) a white pigment, preferably TiO₂; advantageously with an average particle size of 20 to 80 µm, preferably of 30 to 70 *µ*m and particularly of 40 to 60 *µ*m;
e.) sweetening agents, preferably acesulpam-K and/or neohesperidine and/or sodium cyclamate and/or sodium saccharin, particularly sodium saccharin;
f.) NaOH, in order to adjust the pH;
g.) colorants;
h.) flavourings;
i.) water.

7. A dental hygiene product according to any one of claims 1 to 6, characterised in that contact occurs between the two said phases in the form of at least two superimposed strips or in the form of a coaxial double or multiple length.

8. A dental hygiene product according to claim 7, characterised in that the acidic phase forms the core of a double or multiple length.

## Patentansprüche

1. Zahnhygieneprodukt in Pastenform, das Kohlendioxid freisetzt, enthaltend eine basische Phase, die ein Bicarbonat oder Carbonat enthält, und eine saure Phase, die eine pharmakologisch verträgliche organische oder anorganische Protonendonorkomponente enthält, das in der Form von zwei getrennten Phasen vorliegt, die erst miteinander in Kontakt gebracht werden nachdem das Zahnhygieneprodukt aus dem Spender ausgetreten ist, dadurch gekennzeichnet, daß der Gehalt an Bicarbonat oder Carbonat in der basischen Phase von 10 bis 15 Gew.% ist und daß es Polyphosphat und Xylitol enthält.

2. Zahnhygieneprodukt gemäß Anspruch 1, dadurch gekennzeichnet, daß der Gehalt an Polyphosphat in der basischen Phase von 3 bis 50 Gew.%, bevorzugt 5 bis 40 Gew.% und insbesondere 5 bis 20 Gew.%, ist.

3. Zahnhygieneprodukt gemäß Anspruch 2, dadurch gekennzeichnet, daß der Gehalt an Xylitol in der basischen Phase 1,0 bis 50 Gew.%, bevorzugt 5 bis 40 Gew.% und insbesondere 20 Gew.%, ist.

4. Zahnhygieneprodukt gemäß einem der Ansprüche 1 bis 3, dadurch gekennzeichnet, daß das Bicarbonat oder Carbonat das Bicarbonat eines Alkali- oder Erdalkalimetalls oder eine Mischung derselben ist.

5. Zahnhygieneprodukt gemäß einem der Ansprüche 1 bis 4, dadurch gekennzeichnet, daß die pharmakologisch verträgliche Protonendonorkomponente eine organische oder anorganische Säure, bevorzugt Weinsäure, Zitronensäure, racemische Weinsäure, Bernsteinsäure, Milchsäure, Äpfelsäure oder Orthophosphorsäure oder eine Mischung derselben, insbesondere Weinsäure, Zitronensäure oder Äpfelsäure oder eine Mischung derselben, am bevorzugtesten Weinsäure oder Zitronensäure oder eine Mischung derselben, ist.

6. Zahnhygieneprodukt gemäß einem der Ansprüche 1 bis 5, dadurch gekennzeichnet, daß die saure und/oder die basische Phase zusätzlich in jedem Fall einen oder mehrere der im folgenden aufgeführten Bestandteile a) bis i) enthält:
a) ein feuchtigkeitsspeicherndes Mittel, insbesondere Sorbitol und/oder Glyzerin;
b) ein Verdickungsmittel, bevorzugt Xanthangummi und/oder Hydroxypropylmethylcellulose und/oder Hydroxyethylcellulose und/oder Gummi arabicum und/oder Alginate, Polyacrylate und Tragantgummi oder Mischungen derselben und/oder verdickendes präzipitiertes Kieselsäuregel mit einer durchschnittlichen Teilchengröße von 1 bis 20 *µ*m, bevorzugt von 1 bis 15 *µ*m, und vorteilhaft mit einer spezifischen Oberfläche von 50 bis 250 m²/g, bevorzugt von 100 bis 200 m²/g und insbesondere von 190 m²/g (bestimmt durch die BET-Methode; DIN 66131) ;
insbesondere Xanthangummi und/oder Hydroxypropylmethylcellulose und/oder Hydroxyethylcellulose;
c) ein Material mit einer schleifenden Wirkung, bevorzugt basierend auf SiO₂; insbesondere schleifendes präzipitiertes Kieselsäuregel mit wenig verdickendem Effekt mit einer durchschnittlichen Teilchengröße von 1 bis 20 *µ*m, bevorzugt von 1 bis 15 *µ*m, und vorteilhaft mit einer spezifischen Oberfläche von 50 bis 100 m²/g, bevorzugt von 70 bis 90 m²/g, und insbesondere von 80 m²/g (bestimmt mit der BET-Methode; DIN 66131);
d) ein Weißpigment, bevorzugt TiO₂; vorteilhaft mit einer durchschnittlichen Teilchengröße von 20 bis 80 *µ*m, bevorzugt von 30 bis 70 *µ*m und insbesondere von 40 bis 60 *µ*m;
e) Süßungsmittel, bevorzugt Acesulpam-K und/oder Neohesperidin und/oder Natriumcyclamat und/oder Natriumsaccharin, insbesondere Natriumsaccharin;
f) NaOH, um den pH einzustellen;
g) Farbstoffe;
h) Geschmacksstoffe;
i) Wasser.

7. Zahnhygieneprodukt gemäß einem der Ansprüche 1 bis 6, dadurch gekennzeichnet, daß der Kontakt zwischen den beiden genannten Phasen in der Form von mindestens zwei übereinanderliegenden Streifen oder in Form von einem koaxialem Doppel- oder Mehrfachstück stattfindet.

8. Zahnhygieneprodukt gemäß Anspruch 7, dadurch gekennzeichnet, daß die saure Phase den Kern eines Doppel- oder Mehrfachstücks bildet.

## Revendications

1. Composition d'hygiène dentaire sous forme de pâte, qui libère du dioxyde de carbone et qui contient une phase basique contenant un bicarbonate ou un carbonate, et une phase acide qui contient un composant donneur de protons, organique ou inorganique, pharmacologiquement compatible, qui est sous la forme de deux phases séparées qui sont d'abord mises en contact l'une avec l'autre après que le produit d'hygiène dentaire ait émergé du distributeur, caractérisée en ce la teneur en bicarbonate ou carbonate dans la phase basique est comprise entre 10 et 15 pour cent en poids, et qu'elle contient un polyphosphate et du xylitol.

2. Produit d'hygiène dentaire selon la revendication 1, caractérisé en ce que la teneur en polyphosphate dans la phase basique est comprise entre 3 et 50 pour cent en poids, de préférence entre 5 et 40 pour cent en poids, et en particulier entre 5 et 20 pour cent en poids.

3. Produit d'hygiène dentaire selon la revendication 2, caractérisé en ce que la teneur en xylitol dans la phase basique est comprise entre 1,0 et 50 pour cent en poids, de préférence entre 5 et 40 pour cent en poids, et en particulier, égale à 20 pour cent en poids.

4. Produit d'hygiène dentaire selon l'une quelconque des revendications 1 à 3, caractérisé en ce que le bicarbonate ou le carbonate est un bicarbonate de métal alcalin ou alcalinoterreux, ou un mélange de ceux-ci.

5. Produit d'hygiène dentaire selon l'une quelconque des revendications 1 à 4, caractérisé en ce que le composant donneur de protons pharmacologiquement compatible est un acide organique ou inorganique, de préférence l'acide tartrique, l'acide citrique, l'acide racémique, l'acide succinique, l'acide lactique, l'acide malique ou l'acide orthophosphorique ou un mélange de ceux-ci, en particulier l'acide tartrique, l'acide citrique ou l'acide malique ou un mélange de ceux-ci, le plus préférablement l'acide tartrique ou l'acide citrique ou un mélange de ceux-ci.

6. Produit d'hygiène dentaire selon l'une quelconque des revendications 1 à 5, caractérisé en ce que la phase acide et/ou la phase basique contiennent en plus, un ou plusieurs des constituants énumérés ci-dessous de (a) à (i) dans chaque cas :
(a) un agent de rétention d'humidité, en particulier le sorbitol et/ou le glycérol ;
(b) un agent épaississant, de préférence la gomme xanthane et/ou l'hydroxypropylméthylcellulose et/ou l'hydroxypropyléthylcellulose et/ou la gomme arabique et/ou les alginates, les polyacrylates et la gomme adragante ou les mélanges de ceux-ci et/ou la silice hydratée épaississante précipitée avec une taille moyenne de particules comprise entre 1 et 20 µm, et avantageusement, avec une surface spécifique de 50 à 250 m²/g, de préférence de 100 à 200 m²/g, et en particulier égale à 190 m²/g (déterminée par la technique BET; DIN 66131); et particulièrement la gomme xanthane et/ou l'hydroxypropylméthylcellulose et/ou l'hydroxypropyléthylcellulose ;
(c) un matériau ayant une action abrasive, de préférence à base de SiO₂; en particulier la silice hydratée abrasive précipitée à faible effet épaississant, d'une taille de particules comprise entre 1 et 20 µm, de préférence entre 1 et 15 µm, et avantageusement, avec une surface spécifique de 50 à 100 m²/g, de préférence de 70 à 90 m²/g, et en particulier égale à 80 m²/g (déterminée par la technique BET; DIN 66131);
(d) un pigment blanc, de préférence TiO₂, avantageusement avec une taille de particules comprise entre 20 et 80 µm, de préférence entre 30 et 70 µm et particulièrement entre 40 et 60 µm;
(e) des édulcorants, de préférence l'acésulpam-K et/ou la néohespéridine et/ou le cyclamate de sodium et/ou la saccharine sodique, et particulièrement la saccharine sodique ;
(f) NaOH afin d'ajuster le pH ;
(g) des colorants ;
(h) des arômes ;
(i) de l'eau.

7. Produit d'hygiène dentaire selon l'une quelconque des revendications 1 à 6, caractérisé en ce que le contact se produit entre les deux dites phases sous la forme d'au moins deux bandes superposées ou sous la forme d'une longueur coaxiale double ou multiple.

8. Produit d'hygiène dentaire selon la revendication 7, caractérisé en ce que la phase acide forme le coeur sur une longueur coaxiale double ou multiple.
